# EUROPEAN PATENT APPLICATION

(11) **EP 3 603 554 A1**
(43) Date of publication of application: **05.02.2020**
(21) Application number: 18775604.4
(22) Date of filing: 23.03.2018
(51) Int. Cl.: A61B 18/14, A61M 1/00, A61M 25/00

(54) **FLOW PASSAGE MEMBER AND HEAD FOR ELECTRIC SCALPEL EMPLOYING SAME**

(30) Priority: 29.03.2017 JP 2017065421
(71) Applicant: Kyocera Corporation, Kyoto-shi, Kyoto 612-8501 (JP)
(72) Inventor: KOGA,Muneki, Kyoto-shi Kyoto 612-8501 (JP)
(74) Representative: Viering, Jentschura & Partner mbB Patent- und Rechtsanwälte
(86) International application number: PCT/JP2018/011788
(87) International publication number: WO 2018/181026

(57) **Abstract**

A flow channel member according to the present disclosure has a flow channel. A surface of the flow channel has a reduced valley depth Rvk found from a roughness profile, the reduced valley depth Rvk being less than a reduced peak height Rpk found from the roughness profile.

## Description

### Field

The present disclosure relates to a flow channel member and an electrosurgical knife head using the flow channel member.

### Background

Electrosurgical knives are used in surgery for excision of cartilage, tumors, foreign matter, and the like, from affected parts. An electrosurgical knife includes an electrosurgical knife head having an electrode for flow of high-frequency electric current therethrough. When the electrosurgical knife is used, cartilage, tumors, foreign matter, and the like in affected parts are able to be excised by: physiological saline solution, which serves as conductive fluid, being dropped to the affected parts; and flow of high-frequency electric current to this physiological saline solution from the electrode included in the electrosurgical knife head. The electrosurgical knife head has, used therein, a flow channel member having a flow channel for suction of the physiological saline solution.

For example, in Patent Literature 1, an electrosurgical knife head having an electrode, which has a rod-shaped body and is arranged inside a ceramic tubular body, has been proposed, and this electrosurgical knife head includes an introducing hole for induction auxiliary liquid.

### Citation List

### Patent Literature

Patent Literature 1: Japanese Patent Application Laid-open No. 2002-136526

### Summary

A flow channel member according to the present disclosure has a flow channel. A surface of the flow channel has a reduced valley depth Rvk found from a roughness profile, the reduced valley depth Rvk being less than a reduced peak height Rpk found from the roughness profile.

### Brief Description of Drawings

FIG. 1 is a perspective view schematically illustrating an example of an electrosurgical knife head according to the present disclosure.
FIG. 2 is a sectional view taken along a line ii-ii in FIG. 1.

### Description of Embodiments

When an electrosurgical knife head has a flow channel member, a flow channel of this flow channel member is used for suction of, not only physiological saline solution, but also blood and pieces of tissue excised from an affected part. In the suction of the blood and the pieces of tissue, as clotted blood generated by heating and pieces of tissue are accumulated in the flow channel, suction force may start to be reduced. There is thus a demand for suction force of a flow channel member used in an electrosurgical knife head to be difficult to be reduced even when blood and pieces of tissue are sucked therein.

Suction force of a flow channel member according to the present disclosure is difficult to be reduced even when blood and pieces of tissue are sucked therein. The flow channel member and an electrosurgical knife head having the flow channel member used therein will hereinafter be described in detail while reference is made to the drawings.

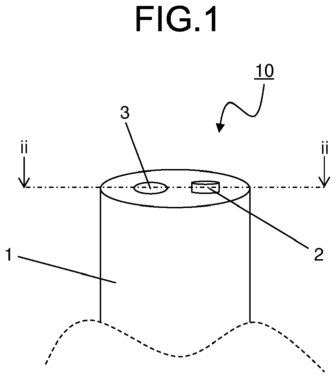

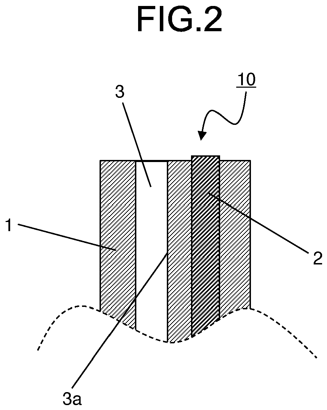

An electrosurgical knife head 10 according to the present disclosure includes, as illustrated in FIG. 1 and FIG. 2, an electrode 2 in a flow channel member 1 having a flow channel 3. The electrosurgical knife head 10 illustrated in FIG. 1 is of a monopolar type including one electrode 2, but without being limited thereto, the electrosurgical knife head 10 may be of a bipolar type including a plurality of the electrodes 2.

A surface 3a of the flow channel 3 in the flow channel member 1 according to the present disclosure has a reduced valley depth Rvk less than a reduced peak height Rpk, the reduced valley depth Rvk and reduced peak height Rpk being found from a roughness profile.

The reduced valley depth Rvk and reduced peak height Rpk are prescribed by JIS B 0671-2 (2002), and are in accordance with the following definitions. First of all, a straight line with the least slope is determined as an equivalent straight line, the straight line being a secant of a material ratio curve, the secant being drawn with a material portion difference being 40% at a central portion of the material ratio curve, the central portion including 40% of measurement points of a roughness profile. Next, a portion between two height positions where this equivalent straight line intersects the vertical axis at positions where a material portion is 0% and 100% is determined as a roughness core. On the roughness profile, a mean depth of protruding valley portions below the roughness core is the reduced valley depth Rvk. A mean height of protruding peak portions above the roughness core is the reduced peak height Rpk.

By satisfying the above configuration, the mean depth of the valley portions in the flow channel member 1 according to the present disclosure is small, and thus when blood and pieces of tissue are sucked into the flow channel member 1, clotted blood and pieces of tissue are difficult to be accumulated in the valley portions and the suction force is difficult to be reduced.

The surface 3a of the flow channel 3 in the flow channel member 1 according to the present disclosure may have a ratio, Rpk/Rvk, equal to or greater than 1.3, the ratio being between the reduced valley depth Rvk and the reduced peak height Rpk. If such a configuration is satisfied, when blood and pieces of tissue are sucked therein, clotted blood and pieces of tissue are more difficult to be accumulated in the valley portions, and the suction force is thus more difficult to be reduced. When the ratio, Rpk/Rvk, on the surface 3a of the flow channel 3 is equal to or greater than 3.0, the suction force is even more difficult to be reduced.

On the surface 3a of the flow channel 3, the reduced valley depth Rvk may be, for example, equal to or greater than 0.2 µm and equal to or less than 1.4 µm. On the surface 3a of the flow channel 3, the reduced peak height Rpk may be, for example, equal to or greater than 0.7 µm and equal to or less than 2.3 µm.

The surface 3a of the flow channel 3 in the flow channel member 1 according to the present disclosure may have a skewness Rsk that is positive, the skewness Rsk being found from the roughness profile.

The skewness Rsk is prescribed by JIS B 0601 (2013) and is an index indicating a ratio between the peak portions and the valley portions with respect to a mean height in the roughness profile, the mean height serving as a center line. When the skewness Rsk is positive, an area of the valley portions is larger than that of the peak portions. Therefore, if such a configuration is satisfied, since the area of the valley portions is large, when blood and pieces of tissue are sucked, clotted blood and pieces of tissue are more difficult to be accumulated in the valley portions, and the suction force is thus more difficult to be reduced.

The surface 3a of the flow channel 3 in the flow channel member 1 according to the present disclosure may have a ratio, S/RSm, equal to or greater than 0.4 and equal to or less than 0.6, the ratio being between a mean interval S between peaks of the peak portions found from the roughness profile and a mean interval RSm between irregularities found from the roughness profile.

The mean interval S between the peaks of the peak portions is prescribed by JIS B 0601 (1994), and is an index indicating a mean value of intervals between peaks of peak portions adjacent to each other. The mean interval RSm between the irregularities is prescribed by JIS B 0601 (2013), and is an index indicating a mean value of intervals, each of which is an interval between one peak portion and one valley portion adjacent to that peak portion, the interval being the sum of lengths of center lines corresponding to the peak portion and valley portion.

When such a configuration is satisfied, when blood and pieces of tissue are sucked, the blood and pieces of tissue are difficult to be stagnated in the valley portions, clotted blood and pieces of tissue are more difficult to be accumulated in the valley portions, and the suction force is thus more difficult to be reduced.

If the mean interval S between the peaks of the peak portions on the surface 3a of the flow channel 3 in the flow channel member 1 according to the present disclosure is equal to or greater than 5 µm and equal to or less than 18 µm, the suction force is more difficult to be reduced. If the mean interval RSm between the irregularities on the surface 3a of the flow channel 3 in the flow channel member 1 according to the present disclosure is equal to or greater than 15 µm and equal to or less than 40 µm, the suction force is more difficult to be reduced.

The surface 3a of the flow channel 3 in the flow channel member 1 according to the present disclosure may have an arithmetical mean roughness Ra equal to or less than 2 µm, the arithmetical mean roughness Ra being found from the roughness profile.

The arithmetical mean roughness Ra, the skewness Rsk, and the mean interval RSm between the irregularities, of the surface 3a of the flow channel 3 in the flow channel member 1 according to the present disclosure are able to be found by measurement conforming to JIS B 0601 (2013); the mean interval S between the peaks of the peak portions is able to be found by measurement conforming to JIS B 0601 (1994); and the reduced valley depth Rvk and reduced peak height Rpk are able to be found by measurement conforming to JIS B 0671-2 (2002). As measurement conditions, for example, a measurement length may be set at 4.8 mm, a cut-off value may be set at 0.8 mm, and a scanning speed of a stylus having a stylus radius of 2 µm may be set at 1.0 mm/second. At least three points or more on the surface 3a of the flow channel 3 are measured, and an average value thereof may be found.

The flow channel member 1 according to the present disclosure may be formed of any of materials, such as metal and resin, but when the flow channel member 1 is made of ceramic, the flow channel member 1 will be high in heat resistance. In particular, when the ceramic is silicon nitride ceramic, the flow channel member 1 will be high in, not only heat resistance, but also in heat conductivity and mechanical strength. When the flow channel member 1 made of silicon nitride ceramic is used in an electrosurgical knife head, even if heating and cooling are repeated, the flow channel member 1 will be less likely to be damaged and will be high in reliability.

Silicon nitride ceramic is ceramic containing 70% or more by mass of silicon nitride in 100% by mass of all components forming the ceramic. Quality of the material of the flow channel member 1 according to the present disclosure may be checked by the following method. First of all, the flow channel member 1 is measured by use of an X-ray diffractometer (XRD), and a value of 2θ (where 2θ is a diffraction angle) obtained thereby is identified by use of JCPDS cards. Subsequently, by use of inductively coupled plasma (ICP) emission spectrophotometer (ICP) or X-ray fluorescence spectrometer (XRF), quantitative analysis for each component forming the flow channel member 1 is performed. In XRD, if the presence of silicon nitride is confirmed, and a content of silicon nitride (Si₃N₄) converted from a content of silicon (Si) measured by the ICP or XRF is equal to greater than 70% by mass, the flow channel member 1 is made of silicon nitride ceramic.

Described hereinafter is a method of manufacturing the flow channel member 1 according to the present disclosure. The flow channel member 1 made of silicon nitride ceramic will be described as an example.

First of all, silicon nitride (Si₃N₄) powder, which is the main raw material, yttrium oxide (Y₂O₃) powder and aluminum oxide (Al₂O₃) powder, which are sintering additives, are: placed, together with a solvent and balls, into a mill; ground until a predetermined particle size is reached; and slurry is thereby formed. As the sintering additives, other than yttrium oxide (Y₂O₃) powder and aluminum oxide (Al₂O₃) powder; calcium oxide (CaO) powder, ferric oxide (Fe₂O₃) powder, tungsten oxide (WO₃) powder, and/or the like may be added.

Subsequently, after a binder is added to the obtained slurry, granules are made by spray drying with a spray dryer.

Subsequently, these granules, thermoplastic resin, wax, and the like are put into a kneader and are kneaded while being heated, and a green body is thereby obtained. By the obtained green body being put into a pelletizer, pellets serving as a material for injection molding are obtained. Subsequently, the obtained pellets are put into an injection molding machine, injection molding is performed, and a molded body having a through hole, which will become the flow channel 3, is thereby obtained.

In order to obtain this molded body having the through hole, which will become the flow channel 3, a forming die enabling the through hole, which will become the flow channel 3, to be obtained may be manufactured, and this molding die may be installed in the injection molding machine and injection molding may be performed, based on a general injection molding method. Since surface characteristics of a portion of the molding die will be transferred onto the inner surface of the through hole, the portion forming the through hole; for the reduced valley depth Rvk to be made smaller than the reduced peak height Rpk on the surface of the flow channel, the molded body may be made by use of a molding die having surface characteristics corresponding thereto. The same applies when arbitrary values for the skewness Rsk, the mean interval S between the peaks of the peak portions, the mean interval RSm between the irregularities, and the arithmetical mean roughness Ra are to be obtained for the surface of the flow channel.

Subsequently, a sintered body is obtained by sintering of the obtained molded body in a nitrogen gas atmosphere with the maximum temperature being held at 1700°C or more and 1800°C or less for 0.5 hours or more and 4 hours or less. The surface of the sintered body obtained is subjected to barrel polishing, and the flow channel member 1 according to the present disclosure is thereby obtained. Conditions of the sintering change depending on the shape and size of the product, and thus are adjusted as needed.

If a hole for insertion of an electrode therethrough is formed at the time of injection molding, and the electrode 2 is fitted in this hole after the sintering, the electrosurgical knife head 10 including the electrode 2 in the flow channel member 1 is able to be obtained.

The present disclosure is not limited to the above described embodiment, and various modifications, improvements, and the like may be made without departing from the gist of the present disclosure.

### Example 1

Samples (flow channel members) having different reduced valley depths Rvk and reduced peak heights Rpk on surfaces of their flow channels were manufactured and their suction forces were evaluated.

First of all, silicon nitride powder; and yttrium oxide powder, aluminum oxide powder, ferric oxide powder, and tungsten oxide powder, which serve as sintering additives, were prepared. Masses of these different kinds of powder were measured, such that a mixture including 84% by mass of silicon nitride powder, 10% by mass of yttrium oxide powder, 4% by mass of aluminum oxide powder, 1% by mass of ferric oxide powder, and 1% by mass of tungsten oxide powder was obtained; and this mixture was placed, with water added thereto, together with balls, into a mill, was ground and mixed, and was thereby made into slurry.

Subsequently, after a binder was added to this slurry, granulated granules were obtained by spray drying of the slurry with a spray dryer. The obtained granules added with thermoplastic resin and wax were put into a kneader, and were kneaded while being heated, and a green body was thereby obtained. Subsequently, the obtained green body was put into a pelletizer, and pellets serving as a material for injection molding were obtained. These pellets were put into an injection molding machine, and a molded body having a through hole, which would become a flow channel, was obtained.

Surface characteristics of a portion of a molding die installed in the injection molding machine, the portion forming the through hole, were made such that the surface of the flow channel of each sample had the reduced valley depth Rvk and the reduced peak height Rpk listed in Table 1.

Subsequently, this molded body was sintered in a nitrogen gas atmosphere with the maximum temperature being 1750°C and the holding time for the maximum temperature being two hours, and a sintered body was thereby obtained. The obtained sintered body was subjected to barrel polishing, and a sample having a diameter of 3.5 mm and a cylindrical shape was each obtained. The flow channel of each sample had a diameter of 1.5 mm and a cylindrical shape.

For each sample obtained, by use of a contact-type surface roughness tester, the reduced valley depth Rvk and the reduced peak height Rpk on the surface of the flow channel were measured based on JIS B 0671-2 (2002). As measurement conditions, the measurement length was set at 4.8 mm, the cut-off value was set at 0.8 mm, and the scanning speed of the stylus having a stylus radius of 2 µm was set at 1.0 mm/second. Three points on the surface of the flow channel were measured, and an average value thereof was calculated.

Subsequently, the suction force of each sample was evaluated. First of all, a tube was connected to the flow channel of each sample, and a solution having silicone with an average particle diameter of 13 µm mixed in a physiological saline solution added with glycerol for simulation of viscosity of blood was caused to flow to the flow channel of the sample from the tube, the silicone simulating pieces of tissue. The solution discharged through the flow channel of each sample was caused to pass through a particle counter, and the number of particles of silicone in the solution discharged was measured.

The samples were then ranked in order from the one with the largest number of particles of silicone in the discharged solution. That is, the sample with the largest number of particles of silicone ranked first, and the sample with the smallest number of particles of silicone ranked last. The higher the number of particles of silicone in the discharged solution is, the more difficult it is for the silicone to be accumulated in the flow channel and the suction force to be reduced when the silicone simulating pieces of tissue is sucked.

These results are listed in Table 1.

**[Table 1]**

| Sample No. | Rvk (µm) | Rpk (µm) | Rpk/Rvk | Ranking |
|---|---|---|---|---|
| 1 | 0.6 | 2.3 | 3.8 | 1 |
| 2 | 0.7 | 2.1 | 3.0 | 3 |
| 3 | 1.0 | 1.9 | 1.9 | 5 |
| 4 | 1.1 | 1.8 | 1.6 | 6 |
| 5 | 1.4 | 1.8 | 1.3 | 7 |
| 6 | 1.5 | 1.8 | 1.2 | 8 |
| 7 | 1.6 | 1.7 | 1.0 | 9 |
| 8 | 1.6 | 1.6 | 1.0 | 10 |
| 9 | 1.6 | 1.5 | 0.9 | 11 |
| 10 | 0.4 | 0.9 | 2.3 | 4 |
| 11 | 0.2 | 0.7 | 3.5 | 2 |

As listed in Table 1, since the rankings of Sample Nos. 1 to 7, 10, and 11 are high, it has been found that the suction force is difficult to be reduced when the reduced valley depth Rvk is smaller than the reduced peak height Rpk on the surface of the flow channel.

Among Sample Nos. 1 to 7, 10, and 11: since the rankings of Sample Nos. 1 to 5, 10, and 11 are high; it has been found that the suction force is more difficult to be reduced when the ratio, Rpk/Rvk, is equal to or greater than 1.3 on the surface of the flow channel.

### Example 2

Samples having positive and negative skewnesses Rsk on surfaces of their flow channels were manufactured, and their suction forces were evaluated.

The manufacturing method therefor was the same as the manufacturing method for Sample No. 1 in Example 1, except that surface characteristics of a portion of a molding die installed in the injection molding machine, the portion forming the through hole, were modified such that the surface of the flow channel of each sample had the skewness Rsk listed in Table 2. Sample No. 12 is the same as Sample No. 1 in Example 1.

For each sample obtained, the skewness Rsk on the surface of the flow channel was measured. Conditions for the measurement were the same as those for Example 1 based on JIS B 0601 (2013). The suction force of each sample was evaluated by the same method as Example 1.

Results thereof are listed in Table 2. The rankings in Table 2 have been assigned by comparison between only the samples listed in Table 2.

**[Table 2]**

| Sample No. | Rsk | Ranking |
|---|---|---|
| 12 | Negative | 2 |
| 13 | Positive | 1 |

As listed in Table 2, since the ranking of Sample No. 13 is higher than that of Sample No. 12, it has been found that the suction force is more difficult to be reduced when the skewness Rsk is positive on the surface of the flow channel.

### Example 3

Samples having different mean intervals S between peaks of peak portions and different mean intervals RSm between irregularities on surfaces of their flow channels were manufactured, and their suction forces were evaluated.

The manufacturing method therefor was the same as the manufacturing method for Sample No. 13 in Example 2, except that surface characteristics of a portion of a molding die installed in the injection molding machine, the portion forming the through hole, were modified such that the surface of the flow channel of each sample had the mean interval S between peaks of peak portions and mean interval RSm between irregularities both listed in Table 3. Sample No. 14 is the same as Sample No. 13 in Example 2.

For each sample obtained, the mean interval S between peaks of peak portions and the mean interval RSm between irregularities on the surface of the flow channel were measured. Conditions for the measurement were the same as those for Example 1 based on JIS B 0601 (1994) and JIS B 0601 (2013). The suction force of each sample was evaluated by the same method as Example 1.

Results thereof are listed in Table 3. The rankings in Table 3 were assigned by comparison among only the samples listed in Table 3.

**[Table 3]**

| Sample No. | S (µm) | RSm (µm) | S/RSm | Ranking |
|---|---|---|---|---|
| 14 | 3 | 11 | 0.3 | 10 |
| 15 | 4 | 11 | 0.4 | 6 |
| 16 | 5 | 12 | 0.4 | 5 |
| 17 | 6 | 15 | 0.4 | 3 |
| 18 | 10 | 26 | 0.4 | 1 |
| 19 | 15 | 40 | 0.4 | 2 |
| 20 | 18 | 41 | 0.4 | 4 |
| 21 | 20 | 41 | 0.5 | 7 |
| 22 | 25 | 42 | 0.6 | 8 |
| 23 | 30 | 44 | 0.7 | 9 |

As listed in Table 3, since the rankings of Sample Nos. 15 to 22 are high, it has been found that the suction force is more difficult to be reduced when the ratio, S/RSm, is equal to or greater than 0.4 and equal to or less than 0.6 on the surface of the flow channel.

Among Sample Nos. 15 to 22, since the rankings of Sample Nos. 16 to 20 are high, it has been found that reduction in the suction force was able to be kept small when the mean interval S between peaks of peak portions on the surface of the flow channel is equal to or greater than 5 µm and equal to or less than 18 µm.

In addition, among Sample Nos. 16 to 20, since the rankings of Sample Nos. 17 to 19 are high, it has been found that reduction in the suction force was able to be kept smaller when the mean interval RSm between irregularities on the surface of the flow channel is equal to or greater than 15 µm and equal to or less than 40 µm.

### Reference Signs List

- 1: FLOW CHANNEL MEMBER
- 2: ELECTRODE
- 3: FLOW CHANNEL
- 3a: SURFACE
10 ELECTROSURGICAL KNIFE HEAD

## Claims

1. A flow channel member, comprising:
a flow channel, wherein
a surface of the flow channel has a reduced valley depth Rvk less than a reduced peak height Rpk, the reduced valley depth Rvk and reduced peak height Rpk being found from a roughness profile.

2. The flow channel member according to claim 1, wherein the surface of the flow channel has a ratio, Rpk/Rvk, equal to or greater than 1.3, the ratio being between the reduced valley depth Rvk and the reduced peak height Rpk.

3. The flow channel member according to claim 1 or 2, wherein the surface of the flow channel has a positive skewness Rsk found from the roughness profile.

4. The flow channel member according to any one of claim 1 to claim 3, wherein the surface of the flow channel has a ratio, S/RSm, equal to or greater than 0.4 and equal to or less than 0.6, the ratio being between a mean interval S between peaks of peak portions found from the roughness profile and a mean interval RSm between irregularities found from the roughness profile.

5. The flow channel member according to any one of claim 1 to claim 4, wherein the surface of the flow channel has a/the mean interval S between peaks of peak portions of 5 µm and more and 18 µm or less, the mean interval S being found from the roughness profile.

6. The flow channel member according to any one of claim 1 to claim 5, wherein the surface of the flow channel has a/the mean interval RSm between irregularities of 15 µm or more and 40 µm or less, the mean interval RSm being found from the roughness profile.

7. The flow channel member according to any one of claim 1 to claim 6, wherein the flow channel member is made of silicon nitride ceramic.

8. An electrosurgical knife head, comprising:
an electrode in the flow channel member according to any one of claim 1 to claim 7.
